# EUROPÄISCHE PATENTANMELDUNG

(11) **EP 4 850 709 A1**
(43) Veröffentlichungstag der Anmeldung: **09.12.2026**
(21) Anmeldenummer: 26174353.8
(22) Anmeldetag: 24.04.2026
(51) Int. Cl.: A61M 1/00, A61F 9/007

(54) **OPHTHALMOLOGISCHE KASSETTE MIT ZUGENTLASTUNG UND SYSTEM**

(30) Priorität: 03.06.2025 DE 102025121611
(71) Anmelder: Geuder AG, 69126 Heidelberg (DE)
(72) Erfinder: Stegmaier, Lukas, 69226 Nußloch (DE); Prisching, Thomas, 68782 Brühl (DE)
(74) Vertreter: Ullrich & Naumann PartG mbB

(57) **Zusammenfassung**

Die Erfindung betrifft eine ophthalmologische Kassette zum Einschieben oder Einstecken in eine ophthalmische Konsole eines ophthalmischen Systems, umfassend zumindest eine erste Aufnahme für eine lösbar einsetzbare Aspirationsleitung und/oder zumindest eine zweite Aufnahme für eine lösbar einsetzbare Irrigationsleitung sowie ein Gehäuse mit einer Stirnseite, einer Rückseite und einer umlaufenden Wandung zwischen Stirnseite und Rückseite, wobei die erste und/oder die zweite Aufnahme eine Nut zur Aufnahme eines Kopplungselements einer Aspirationsleitung aufweist.

Die Erfindung betrifft weiter ein System aus einer ophthalmologischen Kassette und einer Irrigationsleitung sowie einer Aspirationsleitung.

## Beschreibung

Die Erfindung betrifft eine ophthalmologische Kassette zum Einschieben oder Einstecken in eine ophthalmische Konsole eines ophthalmischen Systems, umfassend zumindest eine erste Aufnahme für eine lösbar einsetzbare Aspirationsleitung und/oder zumindest eine zweite Aufnahme für eine lösbar einsetzbare Irrigationsleitung sowie ein Gehäuse mit einer Stirnseite, einer Rückseite und einer umlaufenden Wandung zwischen Stirnseite und Rückseite.

Die Erfindung betrifft weiter ein System aus einer ophthalmologischen Kassette und einer Aspirationsleitung sowie einer Irrigationsleitung, die in der ersten und der zweiten Aufnahme der ophthalmologischen Kassette anordenbar sind.

Ophthalmologische Kassetten der in Rede stehenden Art sind seit Jahren aus der Praxis bekannt. Lediglich beispielhaft sei dazu auf eine Kassette der Anmelderin verwiesen, wie sie in DE 10 2021 201 040 A1 beschrieben ist. Die bekannte Kassette dient zum Einschieben oder Einstecken in eine ophthalmische Konsole eines ophthalmischen Systems. Die Kassette umfasst ein Gehäuse mit mehreren Anschlüssen zum Anschließen externer Schlauch-/Leitungssysteme an innere Fluidkanäle, wobei es sich dabei um einen inneren Aspirationskanal und/oder einen inneren Irrigationskanal handeln kann.

Bei ophthalmologischen Eingriffen besteht oftmals die Notwendigkeit dem Auge Flüssigkeit zuzuführen (sog. Irrigation) sowie Flüssigkeit aus dem Auge abzuführen bzw. abzusaugen (sog. Aspiration). Die Irrigationsflüssigkeit und die Aspirationsflüssigkeit werden hierbei regelmäßig über eine Konsole zur Verfügung gestellt, die mit den entsprechenden chirurgischen Handgeräten beispielsweise über eine Schlauchverbindung verbunden ist. Beispielsweise wird bei einer Phakoemulsifikation dem Auge Irrigationsflüssigkeit zugeführt, während die getrübte Linse mittels Ultraschalls zerkleinert und die Linsentrümmer zusammen mit der Irrigationsflüssigkeit aus dem Auge abgesaugt werden. Um ein steriles Arbeiten zu ermöglichen, wird die Flüssigkeit über eine ophthalmologische Kassette geführt, die in die Konsole eingebringbar ist, wobei die Kassette bislang nach jedem Eingriff entsorgt wird.

Derartige ophthalmologische Kassetten können eine Irrigations- und eine Aspirationsleitung aufweisen, die entlang der Kassette angeordnet sind. Eine Rollenpumpe kann diese Leitungen walken und so eine Förderung einer Flüssigkeit in den Leitungen ermöglichen. Um einen genauen Volumenstrom zu ermöglichen, kann die Irrigations- und/oder Aspirationsleitung vorgespannt sein.

Nachteilig an dieser Anordnung ist, dass die Vorspannung der Leitungen aufwendig ist und voraussetzt, dass die Leitungen, die aus der Kassette hinausführen auf festgelegte Weise angeordnet sind. Darüber hinaus ist ein Nachteil, dass ein Nutzer bei jedem Wechsel des Schlauchsystems die korrekte Befestigung des Aspirationsbehälters gewährleisten muss. Weiterhin ist ein Nachteil, dass Nutzer während der Operation an dem Schlauchsystem "hängen" bleiben könnte und somit eine große Kraft auf das Schlauchsystem ausüben könnte.

Der vorliegenden Erfindung liegt daher die Aufgabe zugrunde, eine Ophthalmologische Kassette der eingangs genannten Art derart auszugestalten und weiterzubilden, dass eine einfache Vorspannung der Leitungen ermöglicht wird und die Wahrscheinlichkeit einer übermäßigen Kraftausübung auf das innere Schlauchsystem innerhalb der Kassette verringert wird.

Erfindungsgemäß werden die voranstehenden Aufgaben durch die Merkmale des Anspruches 1 gelöst. Danach ist die in Rede stehende ophthalmologische Kassette dadurch gekennzeichnet, dass die erste und/oder die zweite Aufnahme eine Nut zur Aufnahme eines Kopplungselements einer Aspirationsleitung und/oder einer Irrigationsleitung aufweist.

Die voranstehenden Aufgaben werden außerdem durch ein System aus einer ophthalmologischen Kassette gemäß einem der Ansprüche 1 bis 5 und einer Aspirationsleitung und/oder einer Irrigationsleitung, wobei die Aspirationsleitung und/oder Irrigationsleitung in der ersten und/oder der zweiten Aufnahme der ophthalmologischen Kassette anordenbar sind, gelöst.

In erfindungsgemäßer Weise ist zunächst erkannt worden, dass durch das Anordnen einer Nut in der Aufnahme das Kopplungselements der Aspirationsleitung bzw. der Irrigationsleitung formschlüssig in der Aufnahme festgelegt werden kann. Hierdurch wird eine Zugentlastung des Schlauchsystems innerhalb und außerhalb der Kassette ermöglicht.. Somit kann die Irrigationsleitung und/oder die Aspirationsleitung auf einfache Weise vorgespannt werden, ohne dass die Spannung der Irrigationsleitung und/oder Aspirationsleitung auf das Schlauchsystem außerhalb der Kassette übertragen wird. Somit kann das äußere Schlauchsystem frei gewählt werden in Bezug auf Orientierung und Material. Außerdem kann das Kopplungselement auf einfache Weise in der Kassette eingeklipst werden, sodass die Aspirations- und/oder Irrigationsleitung auf einfache Weise vorgespannt werden kann. Hierdurch kann auch verhindert werden, dass ein Nutzer, der an dem äußeren Schlauchsystem oder dem Aspirationsbeutel hängen bleibt, das innere Schlauchsystem abreißt, da durch die Zugentlastung die Kraft nicht auf das innere Schlauchsystem übertragen wird. Außerdem ist ein Vorteil, dass das innere Schlauchsystem schnell und einfach gewechselt werden kann, da ein definierter Einklipspunkt für den Schlauch vorgesehen ist.

Gemäß einer vorteilhaften Ausgestaltung ist die Nut in der umlaufenden Wandung vorgesehen. Insbesondere kann es sich bei der ersten und/oder zweiten Aufnahme um eine Bohrung in der Wandung handeln, wobei die Nut um die erste und/oder zweiten Aufnahme angeordnet ist. Die Höhe der umlaufenden Wandung - definiert als die Strecke zwischen Stirnseite und Rückseite der Kassette - ist insbesondere klein im Vergleich zu der Länge und/oder Breite der Sitrnseite und/oder Rückseite. Diese Wandung kann einen verdickten Bereich aufweisen, in dem die Nut angoerdnet ist. Die Nut ist insbesondere eine Ausnehmung innerhalb der Wandung, die auf einer Vorder- und einer Rückseite der Nut - in Anschlussrichtung der Aspirationsleitung teilweise durch die Wandung begrenzt ist. Das heißt, dass die Wandung eine Bewegung eines Kopplungselement innerhalb der Nut in die Kassette hinein und aus der Kassette hinaus begrenzt. Ein Vorteil hiervon ist, dass die Nut auf einfache Weise vorgesehen werden kann. Ein weiterer Vorteil ist, dass die Nut bauraumeffizient vorgesehen werden kann.

Vorteilhafterweise ist die Aufnahme zylinderförmig ausgebildet und die Nut ist achsensymmetrisch zu einer Mittelachse der ersten und/oder zweiten Aufnahme ausgebildet. Beispielsweise kann die Aufnahme eine zylinderförmige Bohrung innerhalb der Wandung sein, durch die Aspirationsleitung geführt ist. Es ist denkbar, dass die Irrigationsleitung durch eine Analoge zweite Aufnahme geführt ist, insbesondere wobei die zweite Aufnahme ebenfalls eine Nut aufweist. Die Nut kann dabei symmetrisch zu der Mittelachse des Zylinders ausgebildet sein, insbesondere sich auf beiden Seiten der Aufnahme entlang der Wandung erstrecken. Hierdurch kann ein Kopplungselement mit zwei seitlichen Flügeln beidseitig innerhalb der Nut festgelegt werden. Ein Vorteil hiervon ist, dass eine besonders gute Zugentlastung des Schlauchsystems innerhalb der Kassette ermöglicht wird.

Dabei ist es insbesondere von Vorteil, wenn die Kassette einen bogenförmigen Anschlag zur Aufnahme einer Rollenpumpe aufweist, wobei mittels der Rollenpumpe eine Strömung innerhalb der Aspirationsleitung erzeugbar ist. Die Rollenpumpe kann die Förderung des Fluids in der Aspirationsleitung durch Walken der Aspirationsleitung ermöglichen. Die Rollenpumpe kann dabei insbesondere in der Konsole vorgesehen sein, sodass die Rollenpumpe beim Einsatz der Kassette in die Konsole an den Anschlag angenähert werden kann, wodurch die Rollenpumpe in die Aspirationsleitung eingreifen kann. Hierdurch wird eine Vorspannung der Aspirationsleitung erzeugt. Diese Vorspannung kann von dem Kopplungselement in der Nut aufgenommen werden, sodass das Kopplungselement und damit die Aspirationsleitung sicher in der Kassette fixiert werden kann.

Gemäß einer bevorzugten Ausgestaltung der Erfindung definiert die Kassette entlang ihrer maximalen Erstreckung eine Haupterstreckungsachse, wobei die Haupterstreckungsachse parallel zu der Stirnseite und/oder Rückseite ausgebildet ist und wobei die Hauptstreckungsachse parallel zu der Mittelachse der ersten und/oder zweiten Aufnahme ausgebildet ist. Mit anderen Worten verläuft die Aufnahme und somit auch eine in die Aufnahme eingesetzte Aspirations- und/oder Irrigationsleitung annähernd parallel zu der Stirnseite und/der der Rückseite der Kassette. Das heißt, dass die Aspirationsleitung und/oder die Irrigationsleitung an der umlaufenden Wandung in die Kassette einführbar ist, sich durch die Kassette erstreckt und auf einer Gegenseite mit einem Anschluss innerhalb der Kassette verbindbar ist. Auf diese Weise kann gewährleistet werden, dass die Aspirationsleitung und/oder Irrigationsleitung lediglich geringe Umlenkungen und oder Knicke aufweist. Insbesondere kann sich die Aspirationsleitung und/oder die Irrigationsleitung innerhalb der Kassette gerade zwischen der Aufnahme und dem Anschluss der Aspirationsleitung und/oder Irrigationsleitung erstrecken. Ein Vorteil hiervon ist, dass der Verschleiß des inneren Schlauchsystems und der Druckverlust des Fluids innerhalb des Schlauchsystems vermindert werden kann.

Es ist denkbar, dass die Aspirationsleitung und/oder die Irrigationsleitung ein erstes Leitungselement und ein zweites Leitungselement umfasst, wobei das erste Leitungselement in der ophthalmologischen Kassette anordenbar ist und wobei das zweite Leitungselement außerhalb der Kassette anordenbar ist, wobei das erste und das zweite Leitungselement durch ein Kopplungselement verbunden sind. Das Kopplungselement verbindet somit das erste Leitungselement und das zweite Leitungselement und ermöglicht eine fluidische Verbindung zwischen den beiden Leitungselementen. Das erste Leitungselement kann Teil des inneren Schlauchsystems in der Kassette sein und das zweite Leitungselement kann Teil des äußeren Schlauchsystems außerhalb der Kassette sein. Es ist denkbar, dass die Irrigationsleitung analog aufgebaut ist. Somit ist das Kopplungselement das Element, das die Zugspannung aufnimmt. Ein Vorteil hiervon ist, dass die Verbindung zwischen dem zweiten Leitungselement und dem Kopplungselement, die am wahrscheinlichsten von einer Zugspannung beeinträchtigt werden könnte, zugfrei gehalten werden kann. Dabei wirkt die Zugentlastung in beide Richtungen, sodass einerseits das Schlauchsystem in der Kassette frei von Kräften ist, die auf das Schlauchsystem außerhalb der Kassette wirken und umgekehrt wird das Schlauchsystem außerhalb der Kassette nicht durch die Vorspannung des ersten Leitungselements beeinflusst.

Gemäß einer vorteilhaften Ausgestaltung der Erfindung ist das Kopplungselement als ein Luer-Element mit einer umlaufenden Krempe ausgebildet. Ein Luer-Element ist ein genormtes Verbindungsstück zwischen medizinischen Werkstücken, die aufgrund eines kegelförmigen Verbindungselements eine sichere, fluidisch dichte Verbindung ermöglichen kann. Das Luer-Element kann gleichzeitig als Kopplungselement genutzt werden, sodass das Luer-Element innerhalb der Nut angeordnet ist und die fluidische Verbindung zwischen dem Schlauchsystem in der Kassette und dem Schlauchsystem außerhalb der Kassette bereitstellen kann. Durch eine umlaufende Krempe kann das Luer-Element gegen die innere Wandung der Nut anschlagen und so innerhalb der Nut festgelegt werden. Ein Vorteil hiervon ist, dass kein separates Kopplungselement benötigt wird.

Es ist ebenfalls denkbar, dass das Kopplungselement als ein Luer-Element mit zumindest einem, vorzugsweise zwei, seitlich angeordneten Flügeln ausgebildet ist. Die seitlichen Flügel sind insbesondere symmetrisch ausgebildet und ragen beidseitig in die Nut hinein. Ein Vorteil hiervon ist, dass die Anschlagfläche des Kopplungselements an die innere Wand der Nut vergrößert ist, sodass die Flächenpressung innerhalb der Nut verringert ist.

In einer bevorzugten Ausgestaltung der Erfindung ist das Kopplungselement mittels einer Steckverbindung mit dem ersten Leitungselement verbunden und/oder ist das Kopplungselement mittels einer Klebverbindung mit dem zweiten Leitungselement verbunden. Auf diese Weise kann das Kopplungselement einfach von dem ersten Leitungselement entfernt werden, sodass das erste Leitungselement ersetzt werden kann. Ein weiterer Vorteil ist, dass das zweite Leitungselement nicht von dem Kopplungselement abrutschen kann, selbst wenn eine hohe Last an dem zweiten Leitungselement anliegt.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: eine Kassette gemäß einer Ausführungsform der vorliegenden Erfindung.
- Fig. 2: eine Detailansicht der Kassette mit eingesetztem Schlauch gemäß einer Ausführungsform der vorliegenden Erfindung.
- Fig. 3: ein System aus einer Kassette mit einer Irrigations- und einer Aspirationsleitung.

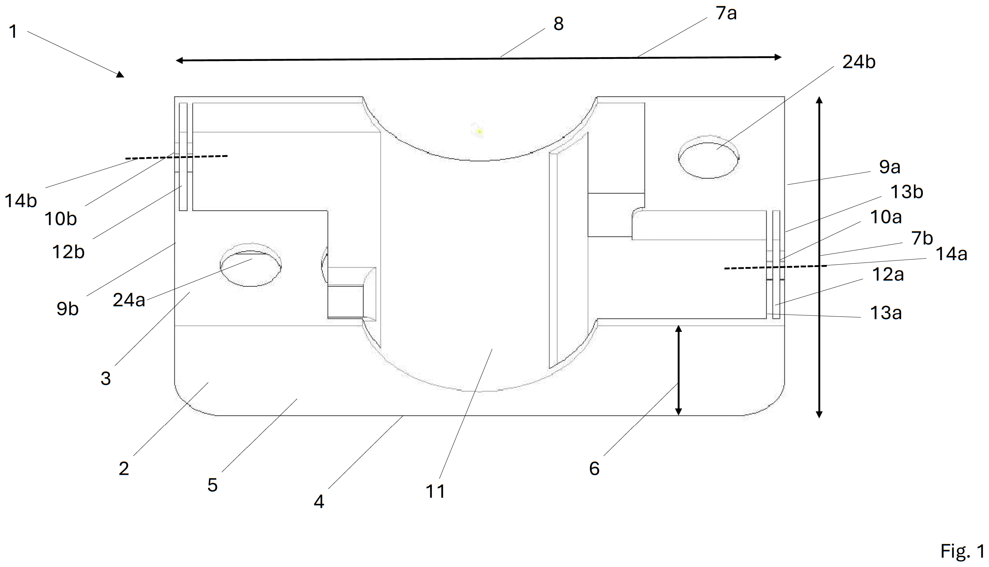

Fig. 1 zeigt eine Kassette gemäß einer Ausführungsform der vorliegenden Erfindung.

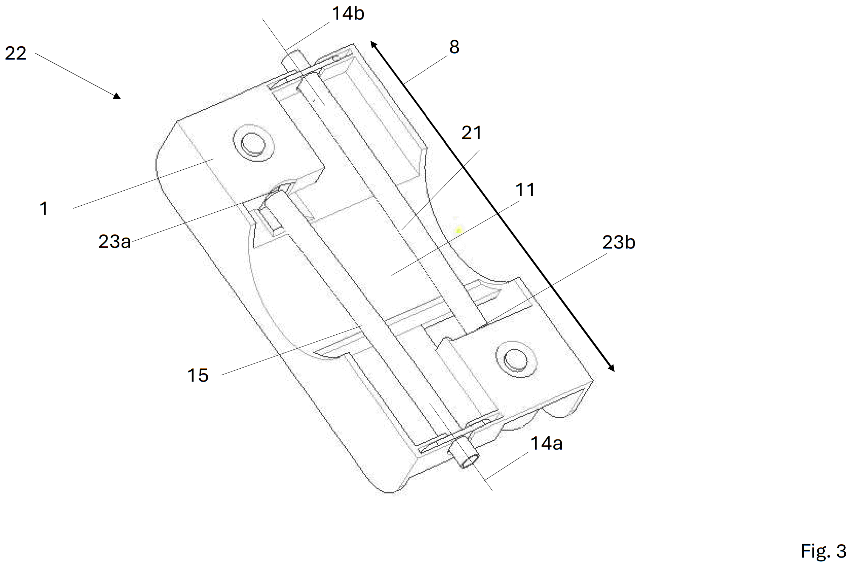

Die Kassette 1 umfasst ein Gehäuse 2 mit einer Stirnseite 3 einer Rückseite 4 (in Figur 1 verdeckt) und einer umlaufenden Wandung 5. Das Gehäuse 2 ist quaderförmig ausgebildet wobei die Höhe 6 der umlaufenden Wandung 5 klein relativ zu der Länge 7a und der Breite 7b der Stirnseite 3 ist. Die Länge 7a der Stirnseite 3 entspricht der maximalen Erstreckung der Kassette 1 und definiert somit die Haupterstreckungsrichtung 8. Die Haupterstreckungsrichtung 8 entspricht dabei der Führung einer Aspirations- und einer Irrigationsleitung (siehe Figur 3) innerhalb der Kassette 1. Auf einer in Haupterstreckungsrichtung 8 vorderen Seite 9a der umlaufenden Wandung 5 des Gehäuses 2 ist eine erste Aufnahme 10a für eine Aspirationsleitung vorgesehen. Im hinteren Teil des Gehäuses 2, das heißt auf der der vorderen Seite 9a abgewandten hinteren Seite 9b ist eine Öffnung 24a vorgesehen, mittels der beispielsweise ein Druck eines Fluids gemessen werden kann. Die Aufnahme 10a ist auf der unteren Seite der Kassette 1 angeordnet. Analog dazu ist auf der oberen Seite der Kassette 1 eine zweite Aufnahme 10b für die Irrigationsleitung vorgesehen, wobei die zweite Aufnahme 10b auf der hinteren Seite 9b angeordnet ist und die entsprechende Öffnung 24b auf der vorderen Seite 9a angeordnet ist. Zwischen der vorderen Seite 9a und der hinteren Seite 9b der umlaufenden Wandung 5 ist eine Anschlagfläche 11 zur Aufnahme einer Rollenpumpe (nicht gezeigt) vorgesehen. Die Rollenpumpe kann an der Anschlagfläche 11 angeordnet werden, sodass die Aspirationsleitung und/oder die Irrigationsleitung durch die Rollenpumpe gewalkt werden kann, wenn die Aspirationsleitung und/oder die Irrigationsleitung entlang der Anschlagfläche 11 angeordnet ist.

Die erste und zweite Aufnahme 10a und 10b sind als Bohrungen innerhalb der umlaufenden Wandung 5 ausgebildet, durch die eine Aspirations- und/oder Irrigationsleitung führbar ist. An den Aufnahmen 10a, 10b ist jeweils eine Nut 12a, 12b vorgesehen. Die Nut erstreckt sich entlang der Wandung 5 senkrecht zu der Haupterstreckungsrichtung 8 - in Figur 1 nach oben und unten. Die Nuten 12a, 12b sind als quaderförmige Ausnehmungen innerhalb der Wandung 5 ausgebildet und jeweils durch eine kassettenseitige Zwischenwand 13a und eine kassettenabgewandte Außenwand 13b teilweise begrenzt. Insbesondere erstrecken sich die Nuten 12a, 12b symmetrisch auf beiden Seiten der Aufnahmen 10a, 10b, d.h. symmetrisch zu einer Mittelachse 14a, 14b der Aufnahmen 10a, 10b.

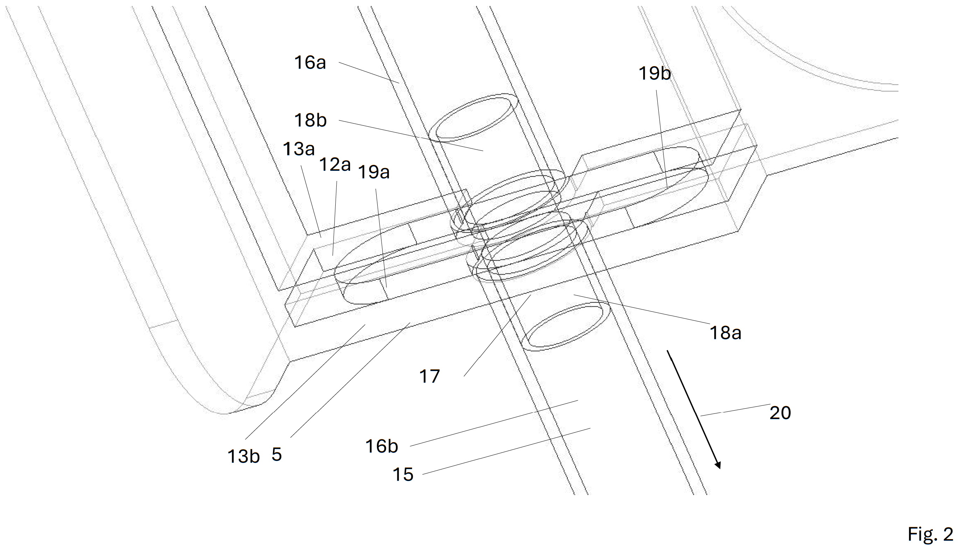

Figur 2 zeigt eine Detailansicht der Kassette mit eingesetztem Schlauch gemäß einer Ausführungsform der vorliegenden Erfindung.

In Figur 2 ist die Aufnahme 10a der Kassette 1 im Detail gezeigt. In der Nut 12a ist eine Aspirationsleitung 15 angeordnet, die sich in die Kassette 1 erstreckt und dort entlang der Anschlagfläche 11 (in Figur 2 nicht gezeigt) für die Rollenpumpe verläuft, sodass die Aspirationsleitung 15 durch die Rollenpumpe gewalkt werden kann. Die Aspirationsleitung 15 umfasst zwei Abschnitte, ein erstes Leitungselement 16a, das innerhalb der Kassette 1 angeordnet ist und ein zweites Leitungselement 16b, das außerhalb der Kassette 1 angeordnet ist. Das erste Leitungselement 16a ist insbesondere Teil der inneren Schlauchsystems und das zweite Leitungselement 16b ist insbesondere Teil des äußeren Schlauchsystems. Die beiden Leitungselemente 16a, 16b sind durch ein Kopplungselement 17, hier in Form einer Luers 17 verbunden, sodass eine durchgehende fluidische Verbindung zwischen den beiden Leitungselementen 16a, 16b entsteht. Der Luer 17 umfasst zwei Anschlüsse 18a, 18b, mit denen der Luer 17 jeweils mit den beiden Leitungselementen 16a, 16b verbunden werden kann. Das erste Leitungselement 16a ist auf den ersten Anschluss 18a aufgesteckt und das zweite Leitungselement 16b ist auf den zweiten Anschluss 18b aufgeklebt.

An dem Luer 17 sind zwei seitliche Flügel 19a, 19b angeordnet, wobei die Dicke der Flügel 19a, 19b derart klein ist, dass sie innerhalb der Nut 12a angeordnet werden können. An dem zweiten Leitungselement 16b kann ein Aspirationsbehälter (nicht gezeigt) angeordnet sein. Dieser kann eine Gewichtskraft entlang des Pfeils 20 erzeugen. Diese Gewichtskraft wird durch die Flügel 19a, 19b des Luers 17 aufgenommen und in die Wandung 5, insbesondere in die Außenwand 13b, eingeleitet. Somit wirkt die Gewichtskraft nicht auf das innere erste Leitungselement 16a - es erfolgt eine Zugentlastung durch den Luer 17. Deswegen kann der erste Anschluss 18a lediglich auf den Luer 17 aufgesteckt sein, ohne dass die Gefahr besteht, dass der Luer 17 von dem ersten Leitungselement 16a durch die Gewichtskraft abgezogen wird. Somit kann das erste Leitungselement 16a einfach nach einer Nutzung entfernt und ersetzt werden.

Der Luer 17 kann in die Nut 12a eingeklickt oder eingeklipst werden, sodass der Luer 17 gleichzeitig als eine Positionierungshilfe dient. Während des Betriebs walkt die Rollenpumpe die Aspirationsleitung 15 und erzeugt so eine Vorspannkraft auf die Aspirationsleitung 15, die entgegen der Pfeilrichtung 20 wirkt. Diese Vorspannkraft drückt den Luer 17 gegen die Zwischenwand 13a, sodass der Luer 17 und somit auch die Aspirationsleitung 15 sicher positioniert ist.

Es ist ebenfalls denkbar, dass die Aspirationsleitung 15 zusätzlich vorgespannt wird, um einen definierten Volumenstrom bereitstellen zu können. Dies ist ebenfalls auf einfache Weise möglich, da die Aspirationsleitung 15 nach dem Vorspannen einfach in die Kassette 1 eingeklipst werden kann.

Die obigen Ausführungen wurden für die Aspirationsleitung 15 gemacht, jedoch kann die Irrigationsleitung (in Figur 2 nicht gezeigt) analog ausgebildet sein.

Figur 3 zeigt ein System aus einer Kassette mit einer Irrigations- und einer Aspirationsleitung.

Gezeigt ist ein System 22 umfassend eine Kassette 1 mit einer eingesetzten Aspirationsleitung 15 und die Irrigationsleitung 21. Die Aspirationsleitung 15 und die Irrigationsleitung 21 verlaufen entlang der Haupterstreckungsrichtung 8 innerhalb der Kassette 1. Insbesondere verlaufen die Aspirations- und Irrigationsleitung 15, 21 gerade von einem Aspirationsanschluss 23a bzw. Irrigationsanschluss 23b bis zu den Aufnahmen 10a, 10b. Durch die Zugentlastung der Luere 17a, 17b wird ein Abreißen der Aspirations- und Irrigationsleitung 15, 21 von dem einem Aspirationsanschluss 23a bzw. Irrigationsanschluss 23b verhindert.

Die Aspirations- und Irrigationsleitung 15, 21 verlaufen dabei insbesondere parallel zu der Hauptstreckungsrichtung 8 und parallel zu der Mittelachse 14a, 14b der Aufnahmen 10a, 10b. Mit anderen Worten verlaufen die Aspirations- und/oder die Irrigationsleitung 15, 21 gerade innerhalb der Kassette 1. Das heißt, dass die Aspirations- und Irrigationsleitung 15, 21 ohne Knicke oder Umlenkungen innerhalb der Kassette 1 verlaufen. Dabei sind die Aspirations- und Irrigationsleitung 15, 21 insbesondere als Schlauchverbindung ausgebildet, die lösbar in der Kassette 1 angeordnet sind.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Kassette
- 2: Gehäuse
- 3: Stirnseite
- 4: Rückseite
- 5: Umlaufende Wandung
- 6: Höhe der Kassette
- 7a, 7b: Länge/Breite der Kassette
- 8: Haupterstreckungsrichtung
- 9a, 9b: Vordere/hintere Seite der umlaufenden Wandung
- 10a: Erste Aufnahme
- 10b: Zweite Aufnahme
- 11: Anschlagfläche
- 12a, 12b: Nuten
- 13a: Zwischenwand
- 13b: Außenwand
- 14a, 14b: Mittelachse
- 15: Aspirationsleitung
- 16a: Erstes Leitungselement
- 16b: Zweites Leitungselement
- 17: Kopplungselement
- 18a, 18b: Anschlüsse
- 19a, 19b: Flügel
- 20: Pfeil
- 21: Irrigationsleitung
- 22: System
- 23a, 23b: Aspirationsanschluss, Irrigationsanschluss
- 24a, 24: Öffnungen

## Patentansprüche

1. Ophthalmologische Kassette (1) zum Einschieben oder Einstecken in eine ophthalmische Konsole eines ophthalmischen Systems, umfassend zumindest eine erste Aufnahme (10a) für eine lösbar einsetzbare Aspirationsleitung (15) und/oder zumindest eine zweite Aufnahme (10b) für eine lösbar einsetzbare Irrigationsleitung (21) sowie ein Gehäuse (2) mit einer Stirnseite (3), einer Rückseite (4) und einer umlaufenden Wandung (5) zwischen Stirnseite (3) und Rückseite (4),
**dadurch gekennzeichnet, dass** die erste und/oder die zweite Aufnahme (10a, 10b) eine Nut (12a, 12b) zur Aufnahme eines Kopplungselements (17) einer Aspirationsleitung (15) und/oder einer Irrigationsleitung (21) aufweist.

2. Ophthalmologische Kassette (1) gemäß Anspruch 1, **dadurch gekennzeichnet, dass** die Nut (12a, 12b) in der umlaufenden Wandung vorgesehen ist.

3. Ophthalmologische Kassette (1) gemäß Anspruch 1 oder 2, **dadurch gekennzeichnet, dass** die erste und/oder die zweite Aufnahme (10a, 10b) zylinderförmig ausgebildet ist und dass die Nut (12a, 12b) achsensymmetrisch zu einer Mittelachse (14a, 14b) der ersten und/oder zweiten Aufnahme (10a, 10b) ausgebildet ist.

4. Ophthalmologische Kassette (1) gemäß einem der Ansprüche 1 bis 3, **dadurch gekennzeichnet, dass** die Kassette (1) einen bogenförmigen Anschlag (11) zur Aufnahme einer Rollenpumpe aufweist, wobei mittels der Rollenpumpe eine Strömung innerhalb der Aspirationsleitung (15) erzeugbar ist.

5. Ophthalmologische Kassette (1) gemäß einem der Ansprüche 1 bis 4, **dadurch gekennzeichnet, dass** die Kassette (1) entlang ihrer maximalen Erstreckung eine Haupterstreckungsachse (8) definiert, wobei die Haupterstreckungsachse (8) parallel zu der Stirnseite (3) und/oder Rückseite (4) ausgebildet ist und wobei die Hauptstreckungsachse (8) parallel zu der Mittelachse (14a, 14b) der ersten und/oder zweiten Aufnahme (10a, 10b) ausgebildet ist.

6. System (22) aus einer ophthalmologischen Kassette (1) gemäß einem der Ansprüche 1 bis 5 und einer Aspirationsleitung (15) und/oder einer Irrigationsleitung (21), wobei die Aspirationsleitung (15) und/oder Irrigationsleitung (21) in der ersten und/oder der zweiten Aufnahme (10a, 10b) der ophthalmologischen Kassette (1) anordenbar sind.

7. System (22) gemäß Anspruch 6, **dadurch gekennzeichnet, dass** zumindest die Aspirationsleitung (15) und/oder die Irrigationsleitung ein erstes Leitungselement (16a) und ein zweites Leitungselement (16b) umfasst, wobei das erste Leitungselement (16a) in der ophthalmologischen Kassette (1) anordenbar ist und wobei das zweite Leitungselement (16b) außerhalb der Kassette (1) anordenbar ist, wobei das erste und das zweite Leitungselement (16a, 16b) durch ein Kopplungselement (17) verbunden sind.

8. System (22) gemäß Anspruch 6 oder 7, **dadurch gekennzeichnet, dass** das Kopplungselement (17) als ein Luer-Element mit einer umlaufenden Krempe ausgebildet ist.

9. System (22) gemäß einem der Ansprüche 6 bis 8, **dadurch gekennzeichnet, dass** das Kopplungselement (17) als ein Luer-Element mit zumindest einem, vorzugsweise zwei, seitlich angeordneten Flügeln (19a, 19b) ausgebildet ist.

10. System (22) gemäß einem der Ansprüche 6 bis 9, **dadurch gekennzeichnet, dass** das Kopplungselement (17) mittels einer Steckverbindung mit dem ersten Leitungselement (16a) verbunden ist und/oder dass das Kopplungselement (17) mittels einer Klebverbindung mit dem zweiten Leitungselement (16b) verbunden ist.
